(19) Europäisches Patentamt

European Patent Office

Office européen des brevets

(11) EP 0 658 540 B1

(12) EUROPÄISCHE PATENTSCHRIFT

(45) Veröffentlichungstag und Bekanntmachung des Hinweises auf die Patenterteilung:
19.03.1997 Patentblatt 1997/12

(51) Int Cl.$^6$: C07C 231/02

(21) Anmeldenummer: 94119479.7

(22) Anmeldetag: 09.12.1994

(54) Verfahren zur Herstellung von hochreinem Isobuttersäureamid

Process for the preparation of high purity isobutylic amide

Procédé pour la préparation de l'amide de l'acide isobutylique avec grande purité

(84) Benannte Vertragsstaaten:
AT CH DE ES FR GB GR IT LI

(30) Priorität: 17.12.1993 DE 4343175

(43) Veröffentlichungstag der Anmeldung:
21.06.1995 Patentblatt 1995/25

(73) Patentinhaber: BASF AKTIENGESELLSCHAFT
67056 Ludwigshafen (DE)

(72) Erfinder:
• Janitschke, Lothar, Dr.
D-67259 Kleinniedesheim (DE)
• Buschmann, Ernst, Dr.
D-67069 Ludwigshafen (DE)

(56) Entgegenhaltungen:
• JOURNAL OF ORGANIC CHEMISTRY., Bd.19, 1954, WASHINGTON US Seiten 623 - 625 G.E. PHILBROOK 'SYNTHESIS OF THE LOWER ALIPHATIC AMIDES'

## Beschreibung

Isobuttersäureamid ist als Therapeutikum für Sichelzellenanämie von Interesse (Experientia 49, 133 (1993), S. P. Perrine, D. V. Faller). Die für die Therapie erforderliche Dosis des Wirkstoffes ist außergewöhnlich hoch. Deshalb wird ein besonders hoher Reinheitsgrad für diese Substanz gefordert.

Die Herstellung von Isobuttersäureamid und verwandter Amide ist in der Literatur mehrfach beschrieben: Houben-Weyl, Methoden der Organischen Chemie, Band VIII, S. 655 ff, N. O. V. Sonntag, Chem. Revs. 52 237 (1953).

Die einfachste Methode zur Herstellung dieser Amide ist die Umsetzung von Säurechloriden mit wäßrigem Ammoniak: O. Aschan, Chem. Ber. 31, 2348 (1898), R. E. Kent, S. M. McElvain, Organic Synthesis, Coll. Vol. III, S. 490 - 492). Die Methode von Aschan liefert jedoch niedrige Ausbeuten. Bessere Ausbeuten werden nach dem Verfahren von Kent-McElvain erzielt.

Bei diesem Verfahren ist allerdings eine mehrstufige Produktisolierung erforderlich. Die Reinheit, die dabei erzielt wird, entspricht nicht den klinischen Anforderungen.

Gute Ausbeuten werden erzielt mit einem wasserfreien Verfahren, das von G. E. Philbrook beschrieben wurde (J. Org. Chem. 19, 623 (1954)).

Dabei wird Isobuttersäurechlorid zu einer Lösung von Ammoniak in Benzol zugetropft. Da sich Ammoniak nur wenig in Benzol löst, wird während der gesamten Zutropfzeit ein Ammoniakgasstrom durch die Lösung geleitet.

Versucht man, das Verfahren zu vereinfachen, indem man das Säurechlorid vorlegt und Ammoniak eingast oder in flüssiger Form zugibt, so bilden sich nach G. E. Philbrook große Mengen des Diamides I.

$$(H_3C)_2CH\text{-}CO\text{-}Cl + NH_3 \rightarrow$$

$$(H_3C)_2CH\text{-}CO\text{-}NH\text{-}CO\text{-}CH(CH_3)_2$$

Bei der Isobuttersäureamid-Herstellung werden Spuren von Isobuttersäurenitril gebildet. Bei der Rückführung des Lösungsmittels Benzol läßt sich das Isobuttersäurenitril nur schwierig destillativ abtrennen, so daß es zur Anreicherung dieses Nebenproduktes bei mehrfacher Verwendung des Benzols kommt. Das hochgiftige Nitril darf jedoch auch nicht in geringsten Spuren in Isobuttersäureamid enthalten sein, welches für medizinische Zwecke verwendet wird.

Es wurde nun ein einfaches Verfahren zur Herstellung von Isobuttersäureamid gefunden.

Gegenstand der Erfindung ist ein Verfahren zur Herstellung von hochreinem Isobuttersäureamid aus Isobuttersäurechlorid und Ammoniak, dadurch gekennzeichnet, daß man zu einer Lösung von Isobuttersäurechlorid in Toluol oder Xylol bei -15° bis 30°C Ammoniak zugibt, die Mischung anschließend erwärmt, in der Wärme das gebildete Ammoniumchlorid abfiltriert und Isobuttersäureamid aus der organischen Lösung auskristallisieren läßt.

Die Zugabe des Ammoniaks erfolgt bei einer Temperatur von etwa -15 bis +30°C. Zweckmäßigerweise beginnt man bei einer Temperatur von etwa -10°C. Bei der Zugabe des Ammoniaks steigt die Temperatur an. Dosiergeschwindigkeit und Kühlung werden so eingestellt, daß die Reaktionstemperatur 30°C nicht übersteigt. Ammoniak kann als Gas in die Lösung des Isobuttersäurechlorids eingeleitet oder in flüssiger Form zugetropft werden. Nach der Ammoniak-Zugabe wird die Reaktionsmischung erwärmt, um alles Isobuttersäureamid in Lösung zu halten.

Zweckmäßigerweise stellt man eine Temperatur von 80°C bis zur Rückflußtemperatur des verwendeten Lösungsmittels ein, vorzugsweise 100°C bis zur Rückflußtemperatur.

Das molare Verhältnis der Mengen von Ammoniak zu Isobuttersäurechlorid, die in die Reaktion eingesetzt werden, liegt bei etwa 2:1 bis 4:1.

Das entstandene Ammoniumchlorid wird zweckmäßig durch Filtration entfernt. Die Lösung wird anschließend auf etwa 30 - 70 % ihres Volumens eingeengt und etwa auf Raumtemperatur oder darunter abgekühlt. Dabei fällt das Isobuttersäureamid aus.

Durch die Verwendung von Toluol bzw. Xylol wird es möglich, Ammoniak in die Lösung von Isobuttersäurechlorid einzuleiten, ohne daß störende Mengen an Diamid entstehen.

Im Gegensatz zum bekannten Verfahren von Philbrook ist bei der Verwendung von Xylol und Toluol die destillative Abtrennung von Isobuttersäurenitrilspuren sehr viel einfacher möglich.

Das neue Verfahren besitzt weiter den Vorteil, daß das entstehende Ammoniumchlorid in Toluol und Xylol völlig unlöslich ist und aus der Reaktionsmischung leicht durch Filtration entfernt werden kann.

Nach dem Einengen der von Ammoniumchlorid befreiten Lösung kristallisiert das Isobuttersäureamid in einer solchen Reinheit aus, daß es ohne weitere Reinigung direkt in Arzneimittel eingearbeitet werden kann.

Das erfindungsgemäß erhaltene Verfahrensprodukt eignet sich besonders gut zur Herstellung von galenischen Applikationsformen, die das Isobuttersäureamid als Wirkstoff entweder alleine oder zusammen mit anderen Wirkstoffen enthalten. Solche Applikationsformen sind beispielsweise Sachets, Sirupe, Trockensirupe.

Das folgende Beispiel beschreibt das erfindungsgemäße Verfahren und eine Verwendungsmöglichkeit für das Produkt.

Beispiel

Eine Lösung von 35 kg Isobuttersäurechlorid in 250 l Toluol wird auf -15° abgekühlt. Innerhalb von 2 h werden 17 kg Ammoniak (flüssig) zudosiert. Die Temperatur steigt dabei auf 30°C an. Man rührt eine Stunde nach, kühlt dabei auf 18°C ab, erwärmt anschließend zum Rückfluß, filtriert das gebildete Ammoniumchlorid ab, wäscht den Filterrückstand viermal mit 25 l Toluol und engt das Filtrat ein, indem man 150 l Toluol abdestilliert. Beim Abkühlen kristallisieren 24,8 kg Isobuttersäureamid (88,2 % Ausbeute) in einer Reinheit von 99,87 % aus.

Um daraus eine Arzneiform herzustellen, werden 4.000 g Isobuttersäureamid, 5.950 g Rohrzucker und 50 g Geschmackstoff miteinander gemischt und jeweils 10 g der Mischung in ein Sachet gefüllt. Die Sachets werden vor Gebrauch in Wasser aufgenommen.

**Patentansprüche**

1. Verfahren zur Herstellung von hochreinem Isobuttersäureamid aus Isobuttersäurechlorid und Ammoniak, dadurch gekennzeichnet, daß man zu einer Lösung von Isobuttersäurechlorid in Toluol oder Xylol bei -15° bis 30°C Ammoniak zugibt die Mischung anschließend erwärmt, in der Wärme das gebildete Ammoniumchlorid abfiltriert und Isobuttersäureamid aus der organischen Lösung auskristallisieren läßt.

**Claims**

1. A process for preparing high-purity isobutyramide from isobutyryl chloride and ammonia, which comprises adding ammonia to a solution of isobutyryl chloride in toluene or xylene at -15° to 30°C, subsequently heating the mixture, removing the ammonium chloride which is formed by hot filtration, and allowing isobutyramide to crystallize out of the organic solution.

**Revendications**

1. Procédé de préparation d'un isobutyramide à haute pureté à partir du chlorure de l'acide isobutyrique et de l'ammoniac, caractérisé par le fait que l'on ajoute de l'ammoniac à une solution du chlorure d'acide isobutyrique dans le toluène ou le xylène à des températures comprises entre -15 et 30°C puis on chauffe le mélange, on filtre à chaud le chlorure d'ammonium formé et on fait cristalliser l'isobutyramide de la solution organique.